# EUROPEAN PATENT APPLICATION

(11) **EP 4 057 295 A1**
(43) Date of publication of application: **14.09.2022**
(21) Application number: 21162038.0
(22) Date of filing: 11.03.2021
(51) Int. Cl.: G16H 40/63, G16H 80/00, G06Q 50/00

(54) **METHOD FOR ESTABLISHING A COMMUNICATION PATH BETWEEN A SUBJECT AND A MEDICAL SYSTEM**

(71) Applicant: Koninklijke Philips N.V., 5656 AG Eindhoven (NL)
(72) Inventor: VOGTMEIER, Gereon, 5656 AE Eindhoven (NL); BUSSA, Nagaraju, 5656 AE Eindhoven (NL); JOHNSON, Mark Thomas, 5656 AE Eindhoven (NL); LEUSSLER, Christoph Günther, 5656 AE Eindhoven (NL); SREENIVASAN, Rithesh, 5656 AE Eindhoven (NL); WUELBERN, Jan Hendrik, 5656 AE Eindhoven (NL); SISODIA, Rajendra Singh, 5656 AE Eindhoven (NL)
(74) Representative: Philips Intellectual Property & Standards

(57) **Abstract**

A method for establishing a communication path between a subject and a medical system (20, 30) in real time, comprising providing, by a data processing unit, a set of communication methods between the subject and the medical system, wherein the communication methods of the set of communication methods differ from each other in terms of the used technology (S10); transmitting, by the data processing unit, at least one test signal from the medical system to the subject via at least one communication method of the set of communication methods (S20); receiving, by the data processing unit, at least one response signal from the subject in response to the test signal (S30); assessing, by the data processing unit, the at least one response signal and deriving an assessing result (S40); in case the assessing result is positive: selecting, by the data processing unit, the at least one communication method of the set of communication methods for establishing the communication path (S50); in case the assessing result is negative: transmitting, by the data processing unit, at least one further test signal from the medical system to the subject via at least one further communication method of the set of communication methods, receiving, by the data processing unit, at least one further response signal from the subject in response to the at least one further test signal,
assessing, by the data processing unit, the at least one further response signal and deriving a further assessing result, in case the further assessing result is positive, selecting the at least one further communication method of the set of communication methods for establishing the communication path (S60).

## Description

### FIELD OF THE INVENTION

The present invention relates to a method for establishing a communication path between a subject and a medical system in real time. Further, the present invention relates to a device for establishing a communication path between a subject and a medical system in real time, a medical imaging system, a medical therapy system, and a computer program element.

### BACKGROUND OF THE INVENTION

Interaction with a subject, such as a communicative interaction, in medical environments is challenging due individual characteristics of the subject, changing environmental conditions, and/or changing process phases in medical systems. Communicative interactions may be carried out, for example, via a loudspeaker, facing the subject, and/or a microphone, provided a response from the subject. Communicative interaction may be important as communication errors may result in an inefficient use of medical systems due to necessary repetitions of process phases or result in dangerous situations for the subject. For example, a stress situation for the subject may arise which may result in a panic attack.

Communicative interaction is crucial in terms of efficiency for the medical system, convenience of the subject and safety of the subject.

### SUMMARY OF THE INVENTION

There may, therefore, be a need to improve a communication between a medical system and a subject undergoing a medical procedure.

The object of the present invention is solved by the subject-matter of the independent claims, wherein further embodiments are incorporated in the dependent claims.

According to a first aspect of the present disclosure a, preferably computer-implemented, method for establishing a communication path between a subject and a medical system in real time is provided. The method comprises the steps of: providing, by a data processing unit, a set of communication methods between the subject and the medical system, wherein the communication methods of the set of communication methods differ from each other in terms of the used technology; transmitting, by the data processing unit, at least one test signal from the medical system to the subject via at least one communication method of the set of communication methods; receiving, by the data processing unit, at least one response signal from the subject in response to the test signal; assessing, by the data processing unit, the at least one response signal and deriving an assessing result; in case the assessing result is positive: selecting, by the data processing unit, the at least one communication method of the set of communication methods for establishing the communication path; in case the assessing result is negative: transmitting, by the data processing unit, at least one further test signal from the medical system to the subject via at least one further communication method of the set of communication methods, receiving, by the data processing unit, at least one further response signal from the subject in response to the at least one further test signal, assessing, by the data processing unit, the at least one further response signal and deriving a further assessing result, in case the further assessing result is positive, selecting the at least one further communication method of the set of communication methods for establishing the communication path.

The term communication path, as used herein, is to be understood broadly, and relates to a communication, e.g. a communication option and/or a communication modality, between a subject and a medical system. The communication path is established by utilizing one or more communication modalities and/or methods. For example, the communication path may comprise an audio communication, a visual communication, a tactile communication, a temperature based communication, or combinations thereof. The communication path may be one way, i.e. only from the medical system towards the subject or vice versa, or in both ways. Optionally, the communication path may be permanent, i.e. may allow a permanent communication. The communication path may vary in the selection of the communication method and/or communication methods over a time due to changing conditions of the subject, the medical system, and/or environmental conditions. The term subject relates in the present case to humans.

The term medical system is to be understood broadly and relates to any system configured to interact with a subject. The medical system may be, for example, a CT system, an X-ray system, an MRT system, a medical therapy system, such as a LINAC, etc.

The term real time is to be understood broadly in the present case and relates to a time period in which the method is capable of establishing a communication path between the subject and the medical system. The time period may be shorter than the reaction time of the medical system. For example, the time period may be less than 1 s, preferably less than 10ms, particularly preferred less than 5 ms. The reaction time may be the time initializing a process phase or a next step in a process phase. The term real time may further mean that method is carried out during ongoing operation of the medical system. The method is therefore a so-called inline process. The term real time may further mean that the method is carried autonomously and continuously. To achieve real time capability, the method is carried out by one or more of a data processing unit, monitoring systems, communication means, which may be connected to each other, via e.g. a hard-wired connection, a bus system, a network connection, such as Ethernet, etc. Further, the method may comprise a continuous feedback loop to transmit a test signal and to receive a response signal and/or a communication signal. Further, the method may comprise a continuous assessing of the response signal (i.e. "check" of one or more communication methods) and a continuous selecting of one or more communication methods. The transmitting, the receiving, the assessing, and the selecting may be carried out in parallel and/or in sequence. In other words, the method may be carried out continuously instead of once.

The term data processing unit is to be understood broadly in the present case and relates to any calculation mean that is configured to carry an algorithm, to receive input data and to provide output data. The data processing unit may be a CPU, a FPGA, a PLC, a workstation. The data processing unit may comprise one hardware element or may distributed on several hardware elements. The data processing unit may be implemented as a virtual machine on superior system (e.g. computing cluster, cloud device). The term communication method is to be understood broadly in the present case and relates to methods configured to establish communication path.

The term communication modality and/or method may comprise an audio communication, a visual communication, a tactile communication, and a temperature based communication. The audio communication may be established by utilizing one or more audio devices, such as a loudspeaker, configured to provide an audio signal from the medical system to the subject or vice versa, and/or a microphone, configured to capture an audio signal from the subject, etc. The visual communication may be established by a visual indication, e.g. by a light signal, a display, etc. The tactile communication, which may also comprise haptic communication, may be established by a tactile and/or haptic actuator, such as a shaker, a hydraulic piston, a pneumatic piston, means for providing an electrical or magnetic field, etc. The temperature based communication may be established by one or more heat and/or cool elements. As such, the communication methods differ in their technical implementation. In other words, the communication methods comprise different technologies compared to each other. The communication method may be arranged in the medical system or adjacent to the medical or be part of the medical system.

The term transmitting via data processing unit means in the present case that the data processing unit controls the communication method (e.g. audio communication method, etc.) to transmit a test signal from the medical system to the subject.

The term test signal is to be understood broadly in the present case and relates to any signal configured to interact with the subject. The test signal may be an audio signal or visual signal. The test signal may comprise a specific signal pattern and signal intensity. The test signal may comprise different signal shapes or patterns, such as a chain tooth pattern, e.g. For example, the test signal may be a sinus signal, triangle signal, Dirac signal etc. The test signal may also comprise a command to act in a specific way, e.g. loudspeaker announcement to inhale or close right eye.

The term response signal is to be understood broadly and relates to any reaction of the subject configured to indicate a response of the subject. The response signal may be provided via a communication path, and may comprise a sound of the subject (e.g. yes), a movement of the subject (e.g. eye, hand sign, leg, change of position), an input of the subject on input device (e.g. button, touchscreen), change of heart beat of the subject, or change of electrical activity of the brain of the subject. The response signal may be initially obtained by a monitoring device such as a microphone, a camera, an EEG, an input device, or a laser interferometer. The response signal may further be transmitted to the data processing unit that is configured to receive and analyze the response signal. The response signal may vary in its intensity, reliability, response or delay time, plausibility. The response signal may be measured during the operating of the medical system.

The term assessing result is to be understood broadly in the present case and relates to a result that compares one or more test signals transmitted from one or more communication methods with one or more response signals. The comparison comprises an analysis of intensity, pattern, frequency with response or delay time of the response signal, intensity of the response signal, plausibility of the response signal. Further, the comparison may comprise a quantitative comparison. The assessing result may comprise a decimal scale from 0.0 to 1.0 with steps of 0.1, wherein 0.0 relates to no response of the subject detected and 1.0 relates to an ideal response of the subject detected. The assessing result may further indicate emergency situations (e.g. in case several communication methods with repeated transmitted test signals lead to no response of the subject which might be an evidence of collapse of the subject).

In other words, the present disclosure is based on the knowledge that a reliable and efficient communication between a subject and a medical system depends on the communication method, in particular the used technology, and signal characteristics of the of the communication method and subject characteristics. In general, each subject may show slight different characteristics and/or conditions on the basis of which a certain communication method and/or communication path is more preferred than another, and/or a certain communication method and/or communication path is more likely to be excluded. For example due to an age, which may affect the subject's ability to hear and/or see, a condition, a mental situation (e.g. stress), and many unknown or unmeasurable characteristics, etc, a certain communication method and/or communication path is more preferred than another. As such, a statistical approach to determine the best communication method between the individual subject and the medical system may only provide inaccurate results. The present method instead tests different communication methods and/or communication paths for each subject before and while operating the medical system to determine what communication method, i.e. communication path, is likely to be sufficient or is preferred to ensure a reliable and efficient communication method between the subject and the medical system, or vice versa. Therefore, the test signal may be transmitted with different signal characteristics (e.g. increasing signal level) and/or utilizing one or more communication methods and/or communication paths to the subject. The subject's reaction, i.e. the response signal, is then monitored and afterwards an analysis of test signal and corresponding response signal reveals whether the communication method is suitable for the individual subject. The order of transmitting test signals via the communication methods can be done based on a sequence of the medical system and current time instance during the operation of the medical system. For instance, if the subject were within a MR bore and during a scan sequence it would be very noisy for him/her to listen to any audio as well as if he/she responds, listening to the response is difficult. The selecting of communication methods may then be reduced to projected display, coded nudging. The response signal may be via finger gestures/ eye blinks etc. The response signal from the subject may be monitored by measuring the reaction time and the plausibility of the response signal within the context of a workflow status and a workflow history.

One key element of the method is that the method is carried out inline the process. This means that during e.g. medical imaging process or between phases of the medical imaging process the method is carried out in real time. Therefore, also changes of subject characteristics (e.g. due increasing stress of the subject) can be identified and result in a selection of another communication method. The method as such continuously tries to find the best communication method for the individual subject. Technical basis for this advantages are high-end communication devices, high-end monitoring devices to measure the subject's response signal and a powerful data processing unit. In sum, the method may lead to an efficient and reliable communication between the individual subject and the medical system. The method may further advantageously detect immediately emergencies (e.g. panic attack or unconsciousness of the subject) and therefore increases the safety of the subject as corrective measures (e.g. stop of the imaging process and alert signals) are initiated automatically. The method may advantageously serve to calibrate before and during the medical procedure continuously the communication methods used in the medical system to establish a reliable communication between the subject and the medical system. The selecting may be based on a neural network.

According to an embodiment, two or more communication methods may be selected for establishing the communication path; and wherein two or more response signals of the of subject in response to test signals of the two or more communication methods are assessed in parallel to assess a plausibility of the two or more response signals of the subject. By selecting two or more communication methods, a redundancy is achieved which advantageously increases the safety of the subject and the efficiency of communication process as unpredictable events during e.g. medical treatment process such as a radiation treatment may only effect the efficiency of one communication method of the two or more selected communication methods. A further example for an unpredictable event may be an action of the subject during a medical imaging process such as breathing, stop breathing, moving a part of the subject etc. Further, by selecting two or more communication methods the plausibility of the response signals of the subject's may be determined by a simple but efficient comparison of the response signals. In case of emergencies also two or more communication channels may be advantageously used to communicate with the subject to deescalate the emergency. The two or more communication channels may also stimulate different human senses (e.g. visual sense and tactile sense or temperature sense and audio sense). This may advantageously increase the reliability of the communication between the subject and the medical system.

In an embodiment, the test signal may comprise different signal levels; and wherein the signal level of the test signal is increased from at least one low level to at least one high level to determine a reception threshold of the subject. The term reception threshold means in the present case the threshold for signal level of a test signal of the subject to which the subject shows a conscious (e.g. moving the right hand) or an unconscious reaction (e.g. change of electrical activity of the brain). By increasing the signal level and measuring the corresponding response signal a different responsiveness of the subject may advantageously be determined. This allows determining the most suitable communication method for the subject. The signal level (e.g. volume of a loudspeaker) is therefore e.g. stepwise increased. This allows further determining a suitable signal level for the communication during the process. For example, the volume is above a reception threshold of the subject but below a certain level which might lead to an inconvenience of the subject. Also in case of hardness of hearing of the subject high reception thresholds may advantageously be determined. In other words, the system emits messages below a priority attention level of the subject but above the non-attention level so that the reaction from the subject is mainly subconscious. In case of no reaction on all communication methods the signal level is increased until at least one reaction is measurable, and an active reaction of the subject indicates the feedback communication. The signal level depends on the communication method and might be the amplitude in the audio system, the light intensity in an optical system, the pressure in a tactile system, etc. The method may be continuously carried out with different signal levels, preferably in a kind of saw-tooth pattern to start with low level, increase the signal in steps of the apparatus allowed digital steps until a response signal is received, select the communication method and use the selected communication method while starting the method step sequence again with lower signal intensity of all communication methods. This ensures the selecting from one communication method to another communication method in case the subject's attention level would drop using the former communication method but might be more responsive using a new communication method. This might be especially important when environmental conditions or workflow steps change (e.g. from subject preparation phase to scanning phase).

In an embodiment, the signal level of the test signal may be increased to a maximum signal level in response to an insufficient response signal and/or an alert signal is emitted in response to the insufficient response signal. The term insufficient response signal relates to a situation, wherein the subject may not react any more to the signals (e.g. deterioration of the physical condition). The term insufficient response signal may also relate conspicuously long delay times (e.g. more than 5s) of the response signals. The term insufficient response signals may also relate to wrong response signals (e.g. wrong behavior of the subject, inhale instead of exhale, merely sound instead of expected words). The increase of the signal level to maximum level is therefore to be understood as an escalation step. In case this escalation step does not solve the problem, an alert signal is emitted as a further escalation step. This may increase the safety of the subject. The alarm signal may further be accompanied by a signal to stop the medical process.

In an embodiment, the selecting of the communication methods may further comprise a choice of a trigger level of the test signal; wherein the trigger level is derived from the reception threshold and the signal levels of the test signal. The term trigger level means in the present case a specific signal level for an individual subject that considers the responsiveness of the individual subject. The trigger level may continuously adapted or maintained after it is determined. The trigger level may be determined before any further medical operating phase of the medical system. The trigger level may serve as specific signal level that enables a sufficient communication within the selected communication method. The trigger levels of different communication methods are normally different to each other. In general, the choice of the trigger level increases the efficacy and the reliability of the communication between the subject and the medical system. The individual trigger levels for subject's reactions may be monitored and logged to check for consistency and malfunction. These logs may be also used to model a communication system to automatically choose the preferred communication method based on workflow steps. This may be done by analyzing and learning from the log files for each of the profiles of the subjects.

In an embodiment, the selecting of the communication methods may be further based on a reaction time between the test signal and the response signal. The term reaction time means the present the time period between transmitted test signal and the received response signal. The reaction time is a measure for the responsiveness of the subject to the one or more communication methods. A short reaction relates to a high responsiveness and vice versa.

In an embodiment, the selecting of the communication methods may be further based on subject data indicative for a responsiveness of the subject and/or on environmental conditions of the subject indicative for the responsiveness of the subject. The term subject data is to be understood broadly in the present case and relates to any data configured to affect the responsiveness of the subject to a communication method. The subject data may comprise age, conditions, records of former used communications methods, sex etc. The consideration of subject data may enable a more reliable selection of the communication method. The term environmental conditions is to be understood broadly in the present case and relates to any conditions configured to affect the responsiveness of the subject. Environmental conditions may comprise temperature, humidity, noise, time of day, vibrations, solar radiation, air flow, spatial position of communication methods in relation to the subject, and monitoring means to measure the response signals. The consideration of environmental conditions may enable a more reliable selection of the communication method. Additional information from the subject record, the subject's profile or other information may be used to pre-select the communication methods. Also, data from subjects population and statistics may be used for selecting the communication method.

The subject may be subjected to a communication method test in the medical preparation stage to check its response levels to various communication methods. For instance, subjects with visual/hearing difficulties would respond better to nudging/touch. Subjects with spectacles may need to remove them and would not be able to respond to projected displays. The subject's comfort with a communication method may also be an input for selecting the communication method. The subject may also be educated and evaluated on communication methods and response signals to improve workflow.

In an embodiment, in case the assessing result is negative and wherein the assessing result indicates an emergency, the at least one further communication method may be selected immediately to establish a communication between the subject and the medical system and/or a control signal is generated, by the data processing unit, to control stopping an operation of the medical system and/or emitting an emergency signal. The term emergency is to be understood broadly in the present case and relates to situations concerning the subject's mental and/or physical health. For example in case several communication methods with repeated transmitted test signals lead to no response of the subject. This may be a hint that the condition of the subject significantly decreased. In such a situation, the second and/or further communication method is immediately selected to establish a communication between the subject and the medical system without transmitting a test signal. In case that the assessing result reveals that the subject is collapsed (e.g. no response with all communications method or detecting calls for) a control signal is generated to control the stopping of the operation of the medical system. The term emergency signal means in the present case a signal configured to notify staff or the like to take care of the subject immediately. In sum, this may increase the safety of the subject. In other words, in case of "no feedback" or no "appropriate reaction" an escalation procedure may start. In this case the signal levels of the former selected communication may be increased to establish a communication path. In an intermediate stage, before sounding of an alarm, more than one communication method is used simultaneously to attract an attention of the subject. Two communication methods may address different sensorial systems of the subject, for example one addressing the audio system whilst the other addresses the optical sense or the tactile sense of the subject. Further good combinations may be the combination of tactile and optical, optical and heat response, audio and heat response, and heat response and tactile.

In an embodiment, the transmitting of test signal via the two or more communications methods may be performed coherently with a repeat frequency between 0.2 and 2Hz. The repeat frequency range from 0.2 to 2 Hz is the range where humans pay particular attention to a synchronous input to two senses (e.g. visual and audio). As such the repeat frequency may increase the reliability of the communication.

In an embodiment, the communication method may comprise an audio communication method, a visual communication method, a tactile communication method and/or a temperature based communication method. The communication method further comprise electrical triggers. The audio communication method may further comprise audio interfaces including options for NLP. The audio communication may comprise loudspeaker. The visual communication method may comprise displays and projections. The tactile communication may comprise pneumatic pistons, shaker or the like. The temperature based communication method may comprises heater and/or cooling elements. The communication method may further comprises combinations of the above mentioned communication methods.

In an embodiment, the response signal may be derived from a monitoring device comprising a video camera system, an ultrasound system, a radar system, a lidar system, an audio system, an EEG system, a bone conduction system and/or an user interface such as a button, a switch, a touch panel.

In an embodiment, a protocol comprising the one or more selected communication methods may become part of a record of the subject and/or assigned to the subject. The record may be used for future medical procedures but also for other subject supporting technologies in aftercare situations (e.g. at home).

In an embodiment, the protocol from all subjects may be analyzed to identify preferred communication methods per workflow procedure for specific subject's group. This record may help to prioritize the most probable communication method in a prediction and the above described method and also may enable a plausibility check during the procedures.

In an embodiment, selecting the one or more communication method may comprise having a relative delay between the test signals and a smooth ramp up of the test signal at the beginning. The parameters for the delay and ramp up may depend on the situation estimated by the response signals and subject data. An adaptive squelch parameter check for the communication methods may be continuously adapted. A smooth threshold level may be calculated for the methods, so that subject may get clear communication of selected communication method. An information burst signal or tone may inform the subject that another communication method may be selected. A confirm signal at the start and end of a communication may be provided, so that subject clearly knows when communication sequence is finished.

In an embodiment, a fail save mechanism may be provided, wherein various means of communication methods and means to monitor the response signals are used to continuously check uninterrupted information transfer between a remote command center and the medical system. This may be important to distinguish if the communication method is interrupted on a technical level or if it is below the subject's reception threshold. For example, installed cameras can observe in-room/in-bore displays, if information is displayed as anticipated by the remote system. Analogously microphones can monitor the transmission of audible signals.

According to a second aspect of the present disclosure, a device for establishing a communication path between a subject and a medical system in real time is provided. The medical system comprises: a provision unit configured to provide a set of communication methods between the subject and the medical system, wherein the communication methods of the set of communication methods differ from each other in terms of the used technology; a transmitting unit configured to transmit at least one test signal from the medical system to the subject via at least one communication method of the set of communication methods; receiving unit configured to receive at least one response signal from the subject in response to the test signal; an assessing unit configured to assess the at least one response signal and deriving an assessing result; a selecting unit configured to select, in case the assessing result is positive, the at least one communication method of the set of communication methods for establishing the communication path; the transmitting unit configured to transmit, in case the assessing result is negative, at least one further test signal from the medical system to the subject via at least one further communication method of the set of communication methods, the receiving unit configured to receive at least one further response signal from the subject in response to the at least one further test signal, the assessing unit configured to assess the at least one further response signal and deriving a further assessing result, the selecting unit configured to select, in case the further assessing result is positive, the at least one further communication method of the set of communication methods for establishing the communication path. The provision unit, the transmitting unit, the receiving unit, the assessing unit, and the selecting unit may be each separate hardware units or implemented in one hardware unit. The hardware unit may be a CPU, a PLC, a FPGA, a microcontroller, a workstation. The provision unit, the transmitting unit, the receiving unit, the assessing unit, and the selecting unit may also be virtual units that run on CPU, a cloud server or the like. In general, the provision unit, the transmitting unit, the receiving unit, the assessing unit, and the selecting unit relate to the above mentioned data processing unit.

A further aspect of the present disclosure relates to a medical imaging system, comprising: a device as described above, and a medical imaging unit. The medical imaging unit may be a CT, an X-Ray system, a MRT system.

A further aspect of the present disclosure relates to a medical therapy system, comprising: a device as described above, and a medical therapy device. The medical therapy device may be a radiation therapy device or a heat therapy device.

Another aspect of the present disclosure relates to a computer program element, which when executed by a processor is configured to carry out the method as described above, and/or to control a device as described above, and/or to control a system as described above. The computer program element might be stored on a computer unit, which might also be part of an embodiment. This computing unit may be configured to perform or induce performing of the steps of the method described above. Moreover, it may be configured to operate the components of the above described device. The computing unit can be configured to operate automatically and/or to execute the orders of a user. A computer program may be loaded into a working memory of a data processor. The data processor may thus be equipped to carry out the method according to one of the preceding embodiments. This exemplary embodiment of the invention covers both, a computer program that right from the beginning uses the invention and computer program that by means of an update turns an existing program into a program that uses invention. Further on, the computer program element might be able to provide all necessary steps to fulfill the procedure of an exemplary embodiment of the method as described above. According to a further exemplary embodiment of the present invention, a computer readable medium, such as a CD-ROM, USB stick or the like, is presented wherein the computer readable medium has a computer program element stored on it which computer program element is described by the preceding section. A computer program may be stored and/or distributed on a suitable medium, such as an optical storage medium or a solid state medium supplied together with or as part of other hardware, but may also be distributed in other forms, such as via the internet or other wired or wireless telecommunication systems. However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It is noted that the above embodiments may be combined with each other irrespective of the aspect involved. Accordingly, the method may be combined with structural features of the device and/or system of the other aspects and, likewise, the device and the system may be combined with features of each other, and may also be combined with features described above with regard to the method.

These and other aspects of the present invention will become apparent from and elucidated with reference to the embodiments described hereinafter.

### BRIEF DESCRIPTION OF THE DRAWINGS

Exemplary embodiments of the invention will be described in the following drawings.
Fig. 1 shows a schematic view of a device according to a first embodiment of the present disclosure;
Fig. 2 shows a flow chart of a method according to a further embodiment of the present disclosure;
Fig. 3 shows a schematic view of a medical imaging system according to a further embodiment of the present disclosure; and
Fig. 4 shows a schematic view of a medical therapy system according to a further embodiment of the present disclosure.

### DETAILED DESCRIPTION OF EMBODIMENTS

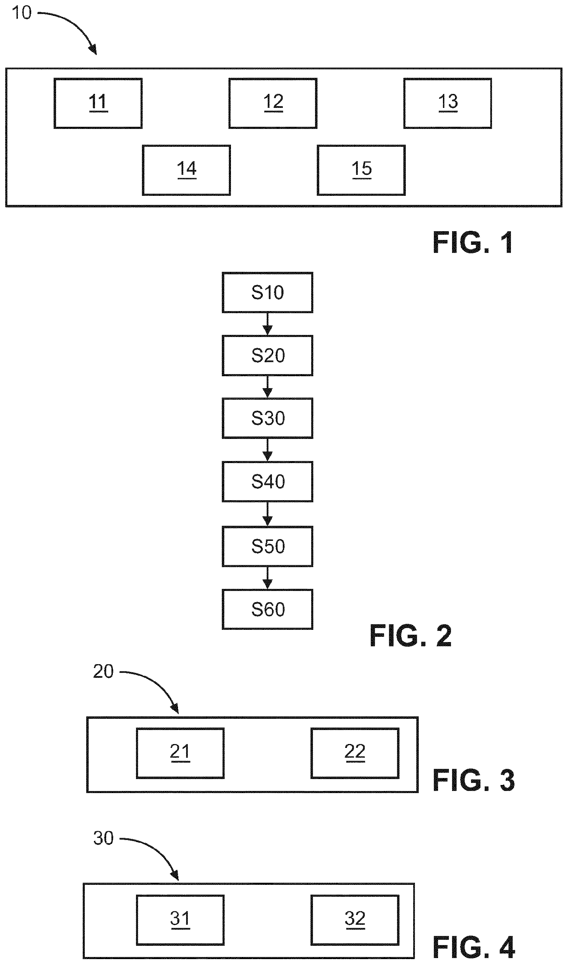

Fig. 1 shows a schematic view of a device 10 according to an exemplary embodiment of the present disclosure. The device 10 is configured to establish a communication path between a subject and a medical system in real time.

The device 10 comprises one or more units or can be functionally divided into several units, interconnected to each other. In particular, the device 10 comprises a provision unit 11 configured to provide a set of communication methods between the subject and the medical system, wherein the communication methods of the set of communication methods differ from each other in terms of the used communication technology. For example, the set of communication method may comprise n different communication methods, wherein n is an integer greater 1, and the n+1 communication method is different to the n-th communication method.

The device 10 further comprises a transmitting unit 12 configured to transmit at least one test signal from the medical system to the subject via at least one communication method of the set of communication methods. For example, the transmitting unit 12 may comprise one or more of a signal generator configured to generate the desired test signal, and a data interface and/or communication interface configured to provide the test signal via a wired and/or wireless connection.

Further, the device 10 comprises a receiving unit 13 configured to receive at least one response signal from the subject in response to the test signal.

The device comprises an assessing unit 14 configured to assess the at least one response signal and deriving an assessing result. The assessment may be implemented by a logic, e.g. in hardware and/or software, configured to compare the test signal and/or response signal to reference, such as a database, a look-up table, or the like, containing one or more thresholds, etc.

The device 10 further comprises a selecting unit 15 configured to select, in case the assessing result is positive, the at least one communication method of the set of communication methods for establishing the communication path. The selecting unit 15 may comprise a logic, or the like, to select the at least one communication method.

For example, the transmitting unit 12 is configured to transmit, in case the assessing result is negative, at least one further test signal from the medical system to the subject via at least one further communication method of the set of communication methods.

Further, the receiving unit 13 is configured to receive at least one further response signal from the subject in response to the at least one further test signal.

The assessing unit 14 is configured to assess the at least one further response signal and deriving a further assessing result, the selecting unit configured to select, in case the further assessing result is positive, the at least one further communication method of the set of communication methods for establishing the communication path.

The provision unit 11, the transmitting unit 12, the receiving unit 13, the assessing unit 14, and the selecting unit 15 may be hardware units separate to each other. Alternatively, the provision unit 11, the transmitting unit 12, the receiving unit 13, the assessing unit 14, and the selecting unit 15 are implemented in one hardware unit, e.g. a workstation.

The first communication method according to the exemplary embodiment, may be an audio communication method, utilizing e.g. a loudspeaker controlled by a suitable driver, and the second communication is a visual communication method, utilizing e.g. a display controlled by a suitable driver. Further, the third and fourth communication method may be a tactile actuator, such as a shaker, and/or a heating actuator, such as a heat element. The response signal of the subject is measured by a touch display, which the subject uses. The test signal shows a chain tooth pattern. The medical system is in the present exemplary embodiment an MRT system. By way of example, some or all of the means configured to provide the communication methods are arranged in a bore of the MRT system.

Fig. 2 shows a flow chart of a method according to a further embodiment of the present disclosure. The method for establishing a communication path between a subject and a medical system in real time comprises several steps, which do not necessarily have to be carried out in the following order.

In first step S10 a set of communication methods between the subject and the medical is provided by the providing unit 11, wherein the communication methods of the set of communication methods differ from each other in terms of the used communication technology. The set of communication methods comprises in the present case a loudspeaker, a display, a shaker, and a heat element.

In a step S20 at least one test signal is transmitted from the medical system to the subject via at least one communication method of the set of communication methods by a transmitting unit 12.

In a step S30 at least one response signal is received from the subject in response to the test signal by the receiving unit 13. The response signal is in the present case emitted from the subject by touching a touch display. This input is received by the receiving unit 13.

In a step S40 the at least one response signal is assessed and an assessing result is derived by an assessing unit 14.

In a step S50 in case the assessing result is positive the at least one communication method of the set of communication methods for establishing the communication path is selected by the selecting unit 15. In the present case, the subject touches the right position in the touch display and the loudspeaker is selected as communication method.

In a step S60 in case the assessing result is negative at least one further test signal from the medical system to the subject via at least one further communication method of the set of communication methods is transmitted by the transmitting unit 12, at least one further response signal is received by the receiving unit 13 from the subject in response to the at least one further test signal, the at least one further response signal is assessed and a further an assessing result is derived by the assessing unit 14, in case the further assessing result is positive, the at least one further communication method is selected of the set of communication methods for establishing the communication path by the selecting unit 15. For example the subject does not touch the right position in the touch display in step S60 the method tests a further communication method for example the display or the shaker and in case the assessing result is positive the display is selected as communication method. In an alternative, also two or more communications method may be selected to establish a communication between the subject and medical system in parallel. In the present exemplary embodiment, a visual communication method, utilizing e.g. a display, and an audio communication method, utilizing e.g. a loudspeaker, are used in parallel.

Fig. 3 shows a schematic view of a medical imaging system according to a further embodiment of the present disclosure. The medical imaging system 20, comprises a device 21 as described above, and a medical imaging unit 22. The medical imaging unit may be a CT, an X-Ray system, a MRT system.

Fig. 4 shows a schematic view of a medical therapy system according to a further embodiment of the present disclosure. The medical therapy system 30 comprises a device 31 as described above, and a medical therapy device 32. The medical therapy device 32 may be a radiation therapy device or a heat therapy device.

In another exemplary embodiment, a computer program or computer program element is provided that is configured to execute the method steps of the method according to one of the preceding embodiments, on an appropriate device or system.

The computer program element might therefore be stored on a data processing unit, which might also be part of an embodiment. This data processing unit may be configured to perform or induce performing of the steps of the method described above. Moreover, it may be configured to operate the components of the above described device and/or system. The computing unit can be configured to operate automatically and/or to execute the orders of a user. A computer program may be loaded into a working memory of a data processor. The data processor may thus be equipped to carry out the method according to one of the preceding embodiments.

Further, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It is noted that embodiments of the present disclosure are described with reference to different subject matter. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and the foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 10,21,31: device
- 11: providing unit
- 12: transmitting unit
- 13: receiving unit
- 14: assessing unit
- 15: selecting unit
- 20: medical imaging system
- 22: medical imaging device
- 30: medical therapy system
- 32: medical therapy device
- S10: providing a set of communication methods
- S20: transmitting a test signal
- S30: receiving a response signal
- S40: assessing test signal and response signal
- S50: selecting a communication method in case of a positive assessing result
- S60: testing another communication method in case of negative assessing result

## Claims

1. A method for establishing a communication path between a subject and a medical system (20, 30) in real time, comprising:
providing, by a data processing unit, a set of communication methods between the subject and the medical system, wherein the communication methods of the set of communication methods differ from each other in terms of the used technology (S10);
transmitting, by the data processing unit, at least one test signal from the medical system to the subject via at least one communication method of the set of communication methods (S20);
receiving, by the data processing unit, at least one response signal from the subject in response to the test signal (S30);
assessing, by the data processing unit, the at least one response signal and deriving an assessing result (S40);
in case the assessing result is positive: selecting, by the data processing unit, the at least one communication method of the set of communication methods for establishing the communication path (S50);
in case the assessing result is negative: transmitting, by the data processing unit, at least one further test signal from the medical system to the subject via at least one further communication method of the set of communication methods,
receiving, by the data processing unit, at least one further response signal from the subject in response to the at least one further test signal,
assessing, by the data processing unit, the at least one further response signal and deriving a further assessing result,
in case the further assessing result is positive, selecting the at least one further communication method of the set of communication methods for establishing the communication path (S60).

2. The method according to claim 1,
wherein two or more communication methods are selected for establishing the communication path; and
wherein two or more response signals of the of subject in response to test signals of the two or more communication methods are assessed in parallel to assess a plausibility of the two or more response signals of the subject.

3. The method according to claim 1 or 2,
wherein the test signal comprises different signal levels; and
wherein the signal level of the test signal is increased from at least one low level to at least one high level to determine a reception threshold of the subject.

4. The method according to claim 3, wherein the signal level of the test signal is increased to a maximum signal level in response to an insufficient response signal and/or an alert signal is emitted in response to the insufficient response signal.

5. The method according to claim 3,
wherein the selecting of the communication methods comprises further a choice of a trigger level of the test signal;
wherein the trigger level is derived from the reception threshold and the signal levels of the test signal.

6. The method according to any one of the preceding claims, wherein the selecting of the communication methods is further based on a reaction time between the test signal and the response signal.

7. The method according to any one of the preceding claims, wherein the selecting of the communication methods is further based on subject data indicative for a responsiveness of the subject and/or on environmental conditions of the subject indicative for the responsiveness of the subject.

8. The method according to any one of the preceding claims, wherein, in case the assessing result is negative and wherein the assessing result indicates an emergency, the at least one further communication method is selected immediately to establish a communication between the subject and the medical system and/or a control signal is generated, by the data processing unit, to control stopping an operation of the medical system and/or emitting an emergency signal.

9. The method according to claim 2, wherein the transmitting of test signal via the two or more communications methods is done coherently with a repeat frequency between 0.2 and 2Hz.

10. The method according to any one of the preceding claims, wherein the communication method comprises an audio communication method, a visual communication method, a tactile communication method and/or a temperature based communication method.

11. The method according to any one of the preceding claims, wherein the response signal is derived from a monitoring device comprising a video camera system, an ultrasound system, a radar system, a lidar system, an audio system, an EEG system, a bone conduction system and/or an user interface such as a button, a switch, a touch panel.

12. A device (10) for establishing a communication path between a subject and a medical system in real time, comprising:
a provision unit (11) configured to provide a set of communication methods between the subject and the medical system, wherein the communication methods of the set of communication methods differ from each other in terms of the used technology;
a transmitting unit (12) configured to transmit at least one test signal from the medical system to the subject via at least one communication method of the set of communication methods;
a receiving unit (13) configured to receive at least one response signal from the subject in response to the test signal;
an assessing unit (14) configured to assess the at least one response signal and deriving an assessing result;
a selecting unit (15) configured to select, in case the assessing result is positive, the at least one communication method of the set of communication methods for establishing the communication path;
the transmitting unit (12) configured to transmit, in case the assessing result is negative, at least one further test signal from the medical system to the subject via at least one further communication method of the set of communication methods,
the receiving unit (13) configured to receive at least one further response signal from the subject in response to the at least one further test signal,
the assessing unit (14) configured to assess the at least one further response signal and deriving a further assessing result,
the selecting unit (15) configured to select, in case the further assessing result is positive, the at least one further communication method of the set of communication methods for establishing the communication path.

13. A medical imaging system (20), comprising:
a device (10, 21) according to claim 12, and
a medical imaging unit (22).

14. A medical therapy system (30), comprising:
a device (10, 31) according to claim 12, and
a medical therapy device (32).

15. A computer program element, which when executed by a processor is configured to carry out the method of any one of the claims 1 to 11, and/or
to control a device according to claim 12, and/or to control a system according to claim 13 or 14.
